# EUROPEAN PATENT APPLICATION

(11) **EP 4 759 266 A1**
(43) Date of publication of application: **17.06.2026**
(21) Application number: 24858548.1
(22) Date of filing: 27.08.2024
(51) Int. Cl.: A61F 2/24

(54) **DELIVERY DEVICE SYSTEM FOR ARTIFICIAL MITRAL VALVE IMPLANT**

(30) Priority: 28.08.2023 CN 202311083575
(71) Applicant: Shanghai Cingular Biotech Corporation, Shanghai 200120 (CN); Yang, Jun, Shanghai 200120 (CN)
(72) Inventor: ZHU, Kaiming, Shanghai 200120 (CN); YANG, Jun, Shanghai 200120 (CN)
(74) Representative: Regimbeau
(86) International application number: PCT/CN2024/114688
(87) International publication number: WO 2025/045012

(57) **Abstract**

Provided is a delivery device system for a prosthetic mitral valve implant. In the delivery device system, a distal movable slider (210) is driven to move axially through a front rotary knob (221) to enable exposure or collapse of a main stent (101) at a target position; and a proximal movable slider (212) is driven to move axially through a rear rotary knob (222) to enable exposure or collapse of an inner stent (102) at the target position. The front rotary knob (221) and the rear rotary knob (222) are controlled to rotate stepwise or synchronously to enable stepwise or synchronous deployment of the main stent (101) and the inner stent (102) with a short interval time, thereby reducing displacement caused by deployment stress.

## Description

This application claims priority to Chinese Patent Application No. 202311083575.X filed with the China National Intellectual Property Administration (CNIPA) on Aug. 28, 2023, the disclosure of which is incorporated herein by reference in its entirety.

### TECHNICAL FIELD

The present application belongs to the technical field of medical devices, relating to an implant delivery system, for example, a delivery device system for a prosthetic mitral valve implant.

### BACKGROUND

Transcatheter mitral valve implantation (TMVI) is an interventional procedure in which a prosthetic valve is used to replace a diseased or abnormal native heart valve. Indications of TMVI include mitral stenosis or mitral regurgitation. A mitral annulus has a saddle-shaped structure in a three-dimensional space and has generally a D-shaped structure in a planar view.

Due to the relatively large size of a mitral valve, a prosthetic mitral valve implant matching the mitral valve also has a relatively large size. At present, most interventional mitral valve implants available on the market have a single cylindrical structure. After being delivered into a human body and deployed and secured at the native mitral annulus, the mitral valve implant provides a relatively large radial support force that may compress the aortic root. A prosthetic valve of a larger size has a larger radial support force, thereby affecting the function of the aortic valve.

For example, CN207804429U discloses a mitral valve prosthesis, a tricuspid valve prosthesis, and a stent thereof. The stent has a collapsed state for delivery and an expanded state for deployment. The stent includes, along the axial direction, an inflow tract, a transition region, and an outflow tract. Opposite ends of the transition region are connected to the inflow tract and the outflow tract respectively. In the expanded state, the inflow tract is located upstream of the outflow tract along a blood flow direction, and the radial stiffness of the inflow tract is smaller than that of the outflow tract and/or the transition region.

In addition, due to the relatively large size, the stent of a single cylindrical design has an excessively large height, and a greater portion of the interventional mitral valve enters the left ventricle, obstructing the left ventricular outflow tract and inducing corresponding diseases.

For example, CN218075337U discloses a mitral valve stent structure including an outer stent and an inner stent connected to the outer stent. The outer stent includes an outer stent body and multiple outer stent connection rings. The outer stent body has a substantially cylindrical hollow structure, and the proximal end of the outer stent body is inwardly folded to form an upper skirt. The multiple outer stent connection rings are circumferentially and uniformly arranged along the inner side of the upper skirt of the proximal end. The inner stent includes an inner stent body having a cylindrical hollow structure and disposed inside the outer stent body and multiple stent connection clasps circumferentially arranged at the distal end of the inner stent body. The proximal inner diameter of the outer stent is greater than the distal outer diameter of the inner stent. The stent connection clasps are snap-fitted with the outer stent connection rings so that the inner stent is suspended on the inner side of the outer stent.

CN216777297U discloses a mitral valve stent including a stent assembly having an outer stent and an inner stent connected to the outer stent. The inner stent includes, from the proximal end to the distal end, a traction portion and a first connecting portion. The proximal end of the traction portion is collapsed. The first connecting portion has a hollow columnar structure. The proximal end of the first connecting portion is connected to the traction portion. The outer stent includes, from the proximal end to the distal end, a second connecting portion and a support portion. The second connecting portion is connected to the distal end of the traction portion. The proximal end of the second connecting portion is collapsed inward. The proximal end of the second connecting portion is drawn out by the proximal end of the traction portion. The support portion has a substantially columnar hollow structure. The first connecting portion is suspended inside the support portion.

In view of the deficiencies of the related art, it is necessary to provide a prosthetic mitral valve implant and a delivery device system therefor that have a stent of a relatively small height, do not affect the aortic root, and can be accurately deployed.

### SUMMARY

An object of the present application is to provide a delivery device system for a prosthetic mitral valve implant. The delivery device system can accurately position the prosthetic mitral valve implant at a corresponding mitral annulus, enabling stepwise or synchronous deployment of a main stent and an inner stent with a short interval time, thereby reducing displacement caused by deployment stress and achieving deployment and fixation.

To achieve this object, the present application uses the following technical solutions.

The present application provides a delivery device system for a prosthetic mitral valve implant. The delivery device system includes a central assembly and a housing assembly.

The central assembly includes an outer sheath, a distal movable slider, a main stent fixing tube, a fixing block, a stepped tube, a proximal movable slider, an inner stent fixing member, and a movable screw rod.

The proximal end of the main stent fixing tube is secured to the fixing block.

The proximal end of the inner stent fixing member is secured to the movable screw rod.

The stepped tube passes through the fixing block. The distal end of the stepped tube is provided with a cavity for accommodating an inner stent in a collapsed state. The proximal end of the stepped tube is secured to the proximal movable slider. A part of the stepped tube is accommodated in the cavity of the main stent fixing tube.

The cavity of the outer sheath is configured to accommodate the main stent fixing tube. The distal end of the outer sheath has a cavity for accommodating a main stent in the collapsed state. The proximal end of the outer sheath is secured to the distal movable slider.

The housing assembly includes a front rotary knob, a rear rotary knob, a threaded rotary knob, and a housing for housing the central assembly.

The front rotary knob is configured to drive the distal movable slider to move axially through rotation.

The rear rotary knob is configured to drive the proximal movable slider to move axially through rotation.

The threaded rotary knob is configured to drive the movable screw rod to move axially through rotation.

The thread pitch of an internal threaded structure of the front rotary knob is greater than or equal to the thread pitch of an internal threaded structure of the rear rotary knob.

In the delivery device system of the present application, the front rotary knob is configured to drive the distal movable slider to move axially to enable exposure or collapse of the main stent fixing tube at a target position; and the rear rotary knob is configured to drive the proximal movable slider to move axially to enable exposure or collapse of the inner stent at the target position. The front rotary knob is rotated to drive the distal movable slider to move axially to enable deployment of the main stent. The rear rotary knob is rotated to drive the proximal movable slider to move axially to enable deployment of the inner stent. The threaded rotary knob is configured to drive the movable screw rod to move axially to enable deployment of the inner stent. The deployment of the inner stent can drive the deployment of the main stent. Thus, the stepwise deployment reduces displacement caused by deployment stress.

In some embodiments, the thread pitch of the internal threaded structure of the front rotary knob is greater than the thread pitch of the internal threaded structure of the rear rotary knob. In the present application, the thread pitch of the internal threaded structure of the front rotary knob is greater than the thread pitch of the internal threaded structure of the rear rotary knob to enable sequential deployment of the main stent and the inner stent.

In some embodiments, the housing assembly also includes a pin assembly.

The pin assembly includes a pin body.

The front rotary knob and the rear rotary knob are each provided with a latching cavity.

The pin body is slidably disposed in the latching cavity.

When it is required to cause the front rotary knob and the rear rotary knob to rotate synchronously, the pin body is inserted into both the latching cavity of the front rotary knob and the latching cavity of the rear rotary knob.

In some embodiments, the distal end of the main stent fixing tube is provided with a first lug groove cooperating with a first lug structure, and the first lug structure is configured to cooperate with the first lug groove to secure the main stent in the collapsed state.

In some embodiments, the number of inner stent fixing members is the same as the number of inner stents, the distal end of the inner stent fixing member is provided with a second lug groove cooperating with a second lug structure, and the second lug structure is configured to cooperate with the second lug groove to secure the inner stent in the collapsed state.

The number of stepped tubes is the same as the number of inner stent fixing members, and each stepped tube is configured to accommodate one inner stent fixing member.

In some embodiments, the front rotary knob has the internal threaded structure, and the internal threaded structure of the front rotary knob is configured to cooperate with an external threaded structure of the distal movable slider; and the front rotary knob is configured to drive the distal movable slider to move axially through rotation.

The rear rotary knob has the internal threaded structure, and the internal threaded structure of the rear rotary knob is configured to cooperate with an external threaded structure of the proximal movable slider; and the rear rotary knob is configured to drive the proximal movable slider to move axially through rotation.

The threaded rotary knob has an internal threaded structure, and the internal threaded structure of the threaded rotary knob is configured to cooperate with an external threaded structure of the movable screw rod; and the threaded rotary knob is configured to drive the movable screw rod to move axially through rotation.

In some embodiments, the prosthetic mitral valve implant applicable to the delivery device system of the present application includes the main stent, at least two inner stents disposed on the inner side of the main stent, and biovalves connected to the at least two inner stents.

Along the axial direction of the main stent, the main stent includes a main stent flange structure at the distal end, a first lug structure at the proximal end, and a main stent body segment connecting the flange structure and the first lug structure.

Along the axial direction of an inner stent, the inner stent includes a second lug structure at the proximal end and an inner stent body segment connected to the second lug structure.

Each inner stent is provided with one biovalve.

A stent fabric is disposed in the gap between the main stent and the at least two inner stents.

In the prosthetic mitral valve implant of the present application, the function of the main stent flange structure is to engage with a cardiac valve annulus during deployment to prevent the stent from disengaging from the cardiac valve annulus during and after deployment. The present application does not limit the structure of the main stent flange structure as long as the corresponding function can be achieved.

The prosthetic mitral valve implant of the present application is configured to replace a native valve by at least two inner stents and corresponding biovalves, reducing the overall valve height and avoiding obstruction of the left ventricular outflow tract. Moreover, while conforming to the mitral annulus, the prosthetic mitral valve implant of the present application can avoid excessive compression of the aortic root, thereby preventing impairment of the normal function of the aortic valve.

In the technical solution of the present application, the term "distal end" refers to the end facing away from an operator, that is, the end adjacent to a patient's heart; and the term "proximal end" refers to the end adjacent to the operator, that is, the end facing away from the patient's heart.

At least two inner stents are disposed on the inner side of the main stent of the prosthetic mitral valve implant in the present application, for example, but not limited to, 2, 3, 4, or 5. Unlisted values within this range are also applicable.

By way of example, the biovalve of the present application includes a stent fabric and at least three leaflets connected to the stent fabric. The stent fabric is disposed on the inner side of the inner stent body segment and is connected to form a closed or non-closed annular shape. The stent fabric is secured to other stent fabrics and to the inner stent body segment by sutures. The at least three leaflets are connected to the stent fabric by sutures. Non-sewed edge portions of the leaflets are coapted in sequence to form a valve structure.

In the present application, the biovalve includes a stent fabric and at least three leaflets connected to the stent fabric. The number of leaflets is at least three, for example, but not limited to, 3, 4, 5, 6, or 8. Unlisted values within this range are also applicable as long as the function of the biovalve can be achieved. In view of structural complexity and costs, in a preferred technical solution, the number of leaflets in the biovalve is 3.

In some embodiments, in the expanded state, the main stent body segment has a height of 6-30 mm.

In some embodiments, in the expanded state, the inner stent body segment has a height of 6-40 mm.

In some embodiments, in the collapsed state, the cross-sectional area of the at least two inner stents is 40-90% of the cross-sectional area of the main stent.

In some embodiments, a barb structure is disposed on the outer side of the main stent body segment.

The prosthetic mitral valve implant of the present application is provided with a barb structure on the outer side of the main stent body segment, facilitating an improvement of the stability of the prosthetic mitral valve implant at the mitral annulus in the expanded state and avoiding adverse effects caused by displacement.

### BRIEF DESCRIPTION OF DRAWINGS

FIG. 1 is a view of components of a prosthetic mitral valve implant with no biovalve and in a collapsed state according to embodiments one to three of the present application.
FIG. 2 is a view of components of a prosthetic mitral valve implant with biovalves and in a collapsed state according to embodiments one to three of the present application.
FIG. 3 is a view of components of a prosthetic mitral valve implant in an expanded state according to embodiments one to three of the present application.
FIG. 4 is an assembled view of a prosthetic mitral valve implant according to embodiments one to three of the present application.
FIG. 5 is a view of the structure of a delivery device system according to embodiments four and five of the present application.
FIG. 6 is an assembled view of a prosthetic mitral valve implant with no biovalve and in a collapsed state in a delivery device system according to embodiments four and five of the present application.
FIG. 7 is an assembled view of a prosthetic mitral valve implant with biovalves and in a collapsed state in a delivery device system according to embodiments four and five of the present application.
FIG. 8 is an exploded view of a prosthetic mitral valve implant with no biovalve and in a collapsed state in a delivery device system according to embodiments four and five of the present application.
FIG. 9 is an exploded view of a prosthetic mitral valve implant with biovalves and in a collapsed state in a delivery device system according to embodiments four and five of the present application.
FIG. 10 is a view of the structure of a central assembly of a delivery device system according to embodiments four and five of the present application.
FIG. 11 is a view of the structure of a housing assembly of a delivery device system according to embodiments four and five of the present application.
FIG. 12 is an assembled view of a central assembly and a housing assembly of a delivery device system according to embodiments four and five of the present application.
FIG. 13 is a view of the action of a pin assembly of a delivery device system according to embodiments four and five of the present application.

### Reference list:

100. prosthetic mitral valve implant; 101. main stent; 1011. first lug structure; 1012. main stent flange structure; 1013. main stent body segment; 1014. barb structure; 102. inner stent; 1021. second lug structure; 1022. inner stent body segment; 103. biovalve; 1031. stent fabric; 1032. leaflet;
201. main stent fixing tube; 2011. first lug groove; 202. inner stent fixing member; 2021. second lug groove; 203. stepped tube; 204. outer sheath; 210. distal movable slider; 211. fixing block; 212. proximal movable slider; 213. movable screw rod; 220. housing; 221. front rotary knob; 2211. first latching cavity; 222. rear rotary knob; 2221. second latching cavity; 223. threaded rotary knob; 224. pin assembly; 2241. pin body

### DETAILED DESCRIPTION

Technical solutions of the present application are further described below through the detailed description. Those skilled in the art are to understand that embodiments described herein are used for a better understanding of the present application and are not to be construed as limitations to the present application.

An embodiment of the present application provides a delivery device system for a prosthetic mitral valve implant. The delivery device system includes a central assembly and a housing assembly.

The central assembly includes an outer sheath, a distal movable slider, a main stent fixing tube, a fixing block, a stepped tube, a proximal movable slider, an inner stent fixing member, and a movable screw rod.

The proximal end of the main stent fixing tube is secured to the fixing block.

The proximal end of the inner stent fixing member is secured to the movable screw rod.

The stepped tube passes through the fixing block. The distal end is provided with a cavity for accommodating an inner stent in a collapsed state. The proximal end is secured to the proximal movable slider. A part of the stepped tube is accommodated in the cavity of the main stent fixing tube.

The cavity of the outer sheath is configured to accommodate the main stent fixing tube. The distal end has a cavity for accommodating a main stent in the collapsed state. The proximal end is secured to the distal movable slider.

The housing assembly includes a front rotary knob, a rear rotary knob, a threaded rotary knob, and a housing for housing the central assembly.

The front rotary knob is configured to drive the distal movable slider to move axially through rotation.

The rear rotary knob is configured to drive the proximal movable slider to move axially through rotation.

The threaded rotary knob is configured to drive the movable screw rod to move axially through rotation.

The thread pitch of the internal threaded structure of the front rotary knob is greater than or equal to the thread pitch of the internal threaded structure of the rear rotary knob.

In the delivery device system of the present application, the front rotary knob is configured to drive the distal movable slider to move axially to enable exposure or collapse of the main stent fixing tube at a target position; and the rear rotary knob is configured to drive the proximal movable slider to move axially to enable exposure or collapse of the inner stent at the target position. The front rotary knob and the rear rotary knob in the present application are controlled to rotate stepwise or synchronously to enable stepwise or synchronous deployment of the main stent and the inner stent with a short interval time, thereby reducing displacement caused by deployment stress.

In some embodiments, the thread pitch of the internal threaded structure of the front rotary knob is greater than the thread pitch of the internal threaded structure of the rear rotary knob. In the present application, the thread pitch of the internal threaded structure of the front rotary knob is greater than the thread pitch of the internal threaded structure of the rear rotary knob, achieving sequential deployment of the main stent and the inner stent.

In some embodiments, the housing assembly also includes a pin assembly.

The pin assembly includes a pin body.

The front rotary knob and the rear rotary knob are each provided with a latching cavity.

The pin body is slidably disposed in the latching cavity.

When it is required to cause the front rotary knob and the rear rotary knob to rotate synchronously, the pin body is inserted into both the latching cavity of the front rotary knob and the latching cavity of the rear rotary knob.

In some embodiments, the distal end of the main stent fixing tube is provided with a first lug groove cooperating with a first lug structure, and the first lug structure is configured to cooperate with the first lug groove to secure the main stent in the collapsed state.

In some embodiments, the number of inner stent fixing members is the same as the number of inner stents, the distal end of the inner stent fixing member is provided with a second lug groove cooperating with a second lug structure, and the second lug structure is configured to cooperate with the second lug groove to secure the inner stent in the collapsed state.

The number of stepped tubes is the same as the number of inner stent fixing members, and each stepped tube is configured to accommodate one inner stent fixing member.

In some embodiments, the front rotary knob has the internal threaded structure, and the internal threaded structure of the front rotary knob is configured to cooperate with an external threaded structure of the distal movable slider; and the front rotary knob is configured to drive the distal movable slider to move axially through rotation.

The rear rotary knob has the internal threaded structure, and the internal threaded structure of the rear rotary knob is configured to cooperate with an external threaded structure of the proximal movable slider; and the rear rotary knob is configured to drive the proximal movable slider to move axially through rotation.

The threaded rotary knob has an internal threaded structure, and the internal threaded structure of the threaded rotary knob is configured to cooperate with an external threaded structure of the movable screw rod; and the threaded rotary knob is configured to drive the movable screw rod to move axially through rotation.

In some embodiments, the prosthetic mitral valve implant applicable to the delivery device system of the present application includes the main stent, at least two inner stents disposed on the inner side of the main stent, and biovalves connected to the inner stents.

Along the axial direction of the main stent, the main stent includes a main stent flange structure at the distal end, a first lug structure at the proximal end, and a main stent body segment connecting the flange structure and the first lug structure.

Along the axial direction of each inner stent, each inner stent includes a second lug structure at the proximal end and an inner stent body segment connected to the second lug structure.

Each inner stent is provided with one biovalve.

A stent fabric is disposed in the gap between the main stent and the inner stents.

In the prosthetic mitral valve implant of the present application, the function of the main stent flange structure is to engage with a cardiac valve annulus during deployment to prevent the stent from disengaging from the cardiac valve annulus during and after deployment. The present application does not limit the structure of the main stent flange structure as long as the corresponding function can be achieved.

The prosthetic mitral valve implant of the present application is configured to replace a native valve by at least two inner stents and corresponding biovalves, reducing the overall valve height and avoiding obstruction of the left ventricular outflow tract. Moreover, while conforming to the mitral annulus, the non-circular prosthetic mitral valve implant of the present application can avoid compression of the aortic root, thereby preventing impairment of the normal function of the aortic valve.

In the technical solution of the present application, the term "distal end" refers to the end facing away from the operator, that is, the end adjacent to the patient's heart; and the term "proximal end" refers to the end adjacent to the operator, that is, the end away from the patient's heart.

At least two inner stents are disposed on the inner side of the main stent of the prosthetic mitral valve implant in the present application, for example, but not limited to, 2, 3, 4, or 5. Unlisted values within this range are also applicable. Prosthetic mitral valve implants of the related art are mostly cylindrical in shape, resulting in excessive compression of the aortic root. The present application effectively solves this problem by providing at least two inner stents. In view of structural complexity and costs, in a preferred technical solution, two inner stents are disposed on the inner side of the main stent.

Prosthetic mitral valve implants of the related art are mostly cylindrical in shape and include only one valve, leading to excessive compression of the aortic root. In addition, such designs have a relatively large valve stent height, obstructing the left ventricular outflow tract to a certain extent and significantly affecting the intraventricular flow field. By using two or more inner stents, the present application reduces the valve stent height while maintaining an effective opening area. Moreover, the overall valve structure is non-circular, avoiding excessive compression of the aortic root and effectively solving the preceding problem. In view of structural complexity and costs, in a preferred technical solution, two inner stents are disposed on the inner side of the main stent.

The biovalve of the present application includes a stent fabric and at least three leaflets connected to the stent fabric. The stent fabric is disposed on the inner side of the inner stent body segment and is connected to form a closed or non-closed annular shape. The stent fabric is secured to other stent fabrics and to the inner stent body segment by sutures. The at least three leaflets are connected to the stent fabric by sutures. Non-sewed edge portions of the leaflets are coapted in sequence to form a valve structure.

In the present application, the biovalve includes a stent fabric and at least three leaflets connected to the sewing cuff. The number of leaflets is at least three, for example, but not limited to, 3, 4, 5, 6, or 8. Unlisted values within this range are also applicable as long as the function of the biovalve can be achieved. In view of structural complexity and costs, in a preferred technical solution, the number of leaflets in the biovalve is 3.

In some embodiments, in the expanded state, the main stent body segment has a height of 6-30 mm, for example, but not limited to, 6 mm, 8 mm, 10 mm, 12 mm, 15 mm, 16 mm, 18 mm, 20 mm, 25 mm, 28 mm, or 30 mm. Unlisted values within this range are also applicable.

In some embodiments, in the expanded state, the inner stent body segment has a height of 6-40 mm, for example, but not limited to, 6 mm, 10 mm, 15 mm, 20 mm, 25 mm, 30 mm, 35 mm, or 40 mm. Unlisted values within this range are also applicable.

In some embodiments, the at least two inner stents have the same structure and are axially arranged in the same plane.

In some embodiments, in the collapsed state, the cross-sectional area of the at least two inner stents is 40-90% of the cross-sectional area of the main stent, for example, but not limited to, 40%, 50%, 60%, 70%, 80%, or 90%. Unlisted values within this range are also applicable.

In the prosthetic mitral valve implant of the present application, the proximal ends of the at least two inner stents are not connected or secured while the distal ends of the at least two inner stents are connected and secured, and the inner stent body segment is connected and secured to the adjacent main stent body segment.

Such connection and securing include riveting and/or suture knotting. This is not limited by the present application as long as the connection and securing can be achieved.

In some examples, a barb structure is disposed on the outer side of the main stent body segment.

The prosthetic mitral valve implant of the present application is provided with a barb structure on the outer side of the main stent body segment, improving the stability of the prosthetic mitral valve implant at the aorta in the expanded state and avoiding adverse effects caused by displacement.

### Embodiment one

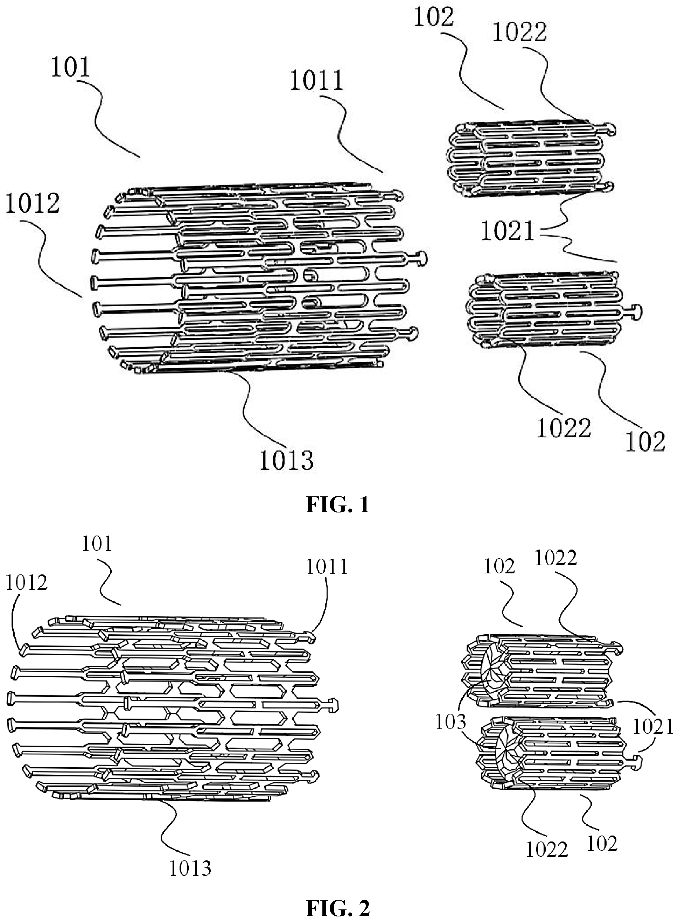

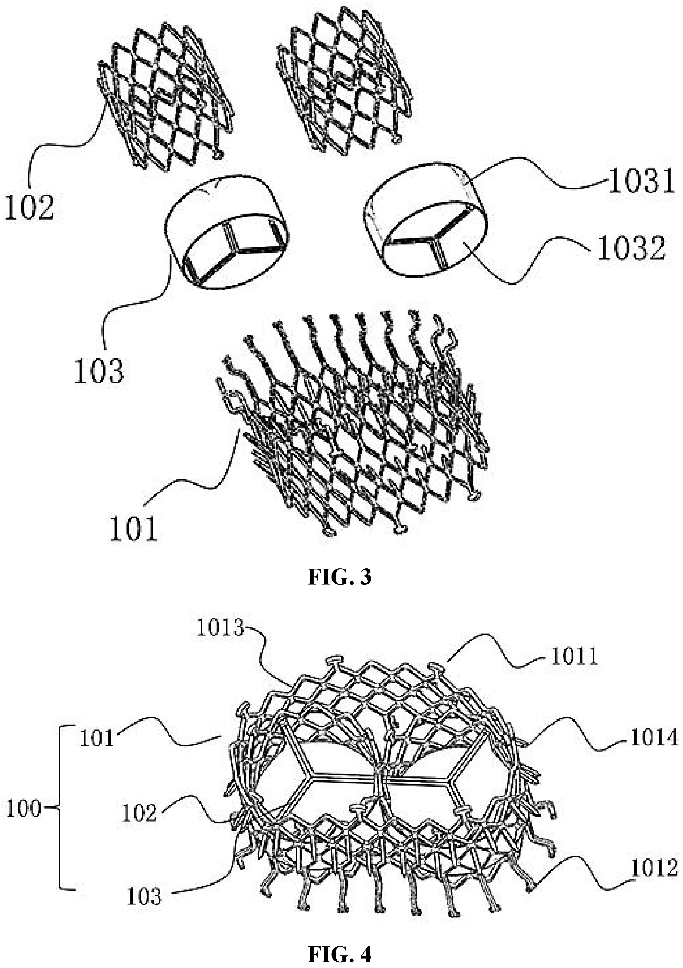

This embodiment provides a prosthetic mitral valve implant 100. As shown in FIG. 1, FIG. 2, FIG. 3, and FIG. 4, the prosthetic mitral valve implant 100 includes a main stent 101, two inner stents 102 disposed on the inner side of the main stent 101, and biovalves 103 connected to the inner stents 102.

The two inner stents 102 have the same structure. The two inner stents 102 are disposed in the same plane. In the collapsed state, the cross-sectional area of the two inner stents 102 is 60% of the cross-sectional area of the main stent 101.

Along the axial direction of the main stent 101, the main stent 101 includes a main stent flange structure 1012 at the distal end, a first lug structure 1011 at the proximal end, and a main stent body segment 1013 connecting the main stent flange structure and the first lug structure 1011.

Along the axial direction of the inner stent 102, the inner stent 102 includes a second lug structure 1021 at the proximal end and an inner stent body segment 1022 connected to the second lug structure 1021.

Each inner stent 102 is provided with one biovalve 103.

The biovalve 103 includes a stent fabric 1031 and three leaflets 1032 connected to the stent fabric 1031. The stent fabric 1031 is disposed on the inner side of the inner stent body segment 1022 and is connected to form a closed annular shape. The stent fabric 1031 is secured to other stent fabrics 1031 and to the inner stent body segment 1022 by sutures. The three leaflets 1032 are connected to the stent fabric 1031 by sutures. Non-sewed edge portions of the leaflets are coapted in sequence to form a valve structure.

The stent fabric 1031 is disposed in the gap between the main stent body segment 1013 and the inner stent body segment 1022.

In the expanded state, the main stent body segment 1013 has a height of 20 mm. In the expanded state, the inner stent body segment 1022 has a height of 20 mm.

A barb structure 1014 is disposed on the outer side of the main stent body segment 1013.

### Embodiment two

This embodiment provides a prosthetic mitral valve implant 100. As shown in FIG. 1, FIG. 2, FIG. 3, and FIG. 4, the prosthetic mitral valve implant 100 includes a main stent 101, two inner stents 102 disposed on the inner side of the main stent 101, and biovalves 103 connected to the inner stents 102.

The two inner stents 102 have the same structure. The two inner stents 102 are disposed in the same plane. In the collapsed state, the cross-sectional area of the two inner stents 102 is 40% of the cross-sectional area of the main stent 101.

Along the axial direction of the main stent 101, the main stent 101 includes a main stent flange structure 1012 at the distal end, a first lug structure 1011 at the proximal end, and a main stent body segment 1013 connecting the main stent flange structure and the first lug structure 1011.

Along the axial direction of the inner stent 102, the inner stent 102 includes a second lug structure 1021 at the proximal end and an inner stent body segment 1022 connected to the second lug structure 1021.

Each inner stent 102 is provided with one biovalve 103.

The biovalve 103 includes a stent fabric 1031 and three leaflets 1032 connected to the stent fabric 1031. The stent fabric 1031 is disposed on the inner side of the inner stent body segment 1022 and is connected to form a non-closed annular shape. The stent fabric 1031 is secured to other stent fabrics 1031 and to the inner stent body segment 1022 by sutures. The three leaflets 1032 are connected to the stent fabric 1031 by sutures. Non-sewed edge portions of the leaflets are coapted in sequence to form a valve structure.

The stent fabric 1031 is disposed in the gap between the main stent body segment 1013 and the inner stent body segment 1022.

In the expanded state, the main stent body segment 1013 has a height of 6 mm. In the expanded state, the inner stent body segment 1022 has a height of 6 mm.

A barb structure 1014 is disposed on the outer side of the main stent body segment 1013.

### Embodiment three

This embodiment provides a prosthetic mitral valve implant 100. As shown in FIG. 1, FIG. 2, FIG. 3, and FIG. 4, the prosthetic mitral valve implant 100 includes a main stent 101, two inner stents 102 disposed on the inner side of the main stent 101, and biovalves 103 connected to the inner stents 102.

The two inner stents 102 have the same structure. The two inner stents 102 are disposed in the same plane. In the collapsed state, the cross-sectional area of the two inner stents 102 is 90% of the cross-sectional area of the main stent 101.

Along the axial direction of the main stent 101, the main stent 101 includes a main stent flange structure 1012 at the distal end, a first lug structure 1011 at the proximal end, and a main stent body segment 1013 connecting the main stent flange structure and the first lug structure 1011.

Along the axial direction of the inner stent 102, the inner stent 102 includes a second lug structure 1021 at the proximal end and an inner stent body segment 1022 connected to the second lug structure 1021.

Each inner stent 102 is provided with one biovalve 103.

The biovalve 103 includes a stent fabric 1031 and three leaflets 1032 connected to the stent fabric 1031. The stent fabric 1031 is disposed on the inner side of the inner stent body segment 1022 and is connected to form a closed annular shape. The stent fabric 1031 is secured to other stent fabrics 1031 and to the inner stent body segment 1022 by sutures. The three leaflets 1032 are connected to the stent fabric 1031 by sutures. Non-sewed edge portions of the leaflets are coapted in sequence to form a valve structure.

A stent fabric 1031 is disposed in the gap between the main stent body segment 1013 and the inner stent body segment 1022.

In the expanded state, the main stent body segment 1013 has a height of 30 mm. In the expanded state, the inner stent body segment 1022 has a height of 40 mm.

A barb structure 1014 is disposed on the outer side of the main stent body segment 1013.

### Embodiment four

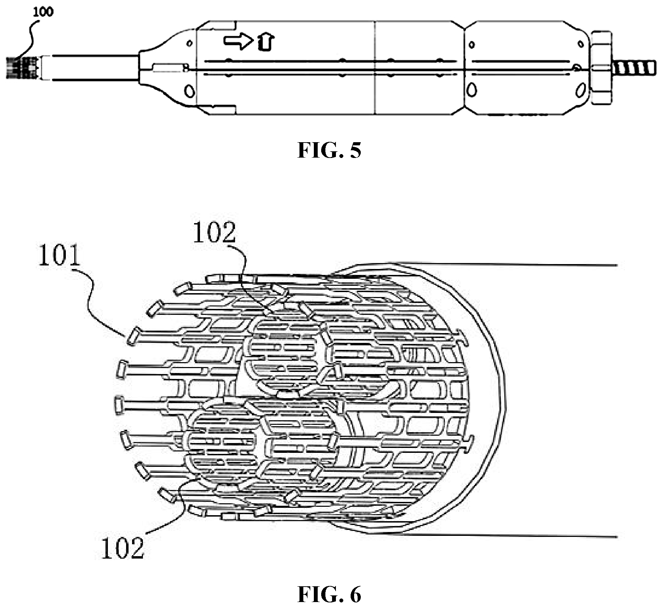

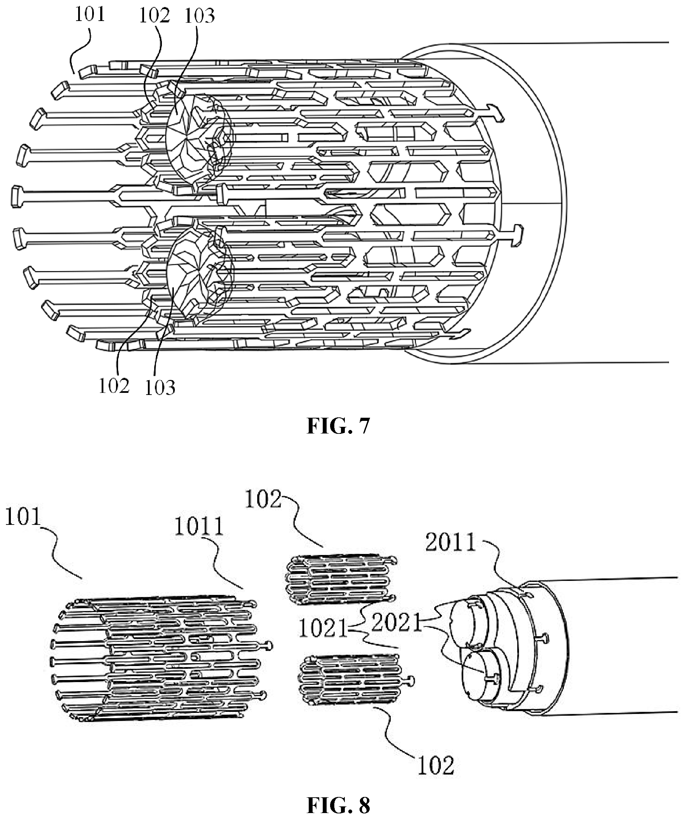

This embodiment provides a delivery device system applicable to the prosthetic mitral valve implant 100 of embodiment one shown in FIG. 5. An assembled view of the delivery device system and the prosthetic mitral valve implant 100 is shown in each of FIGS. 6, 7, 8, and 9.

The delivery device system includes a central assembly and a housing assembly cooperating with the central assembly.

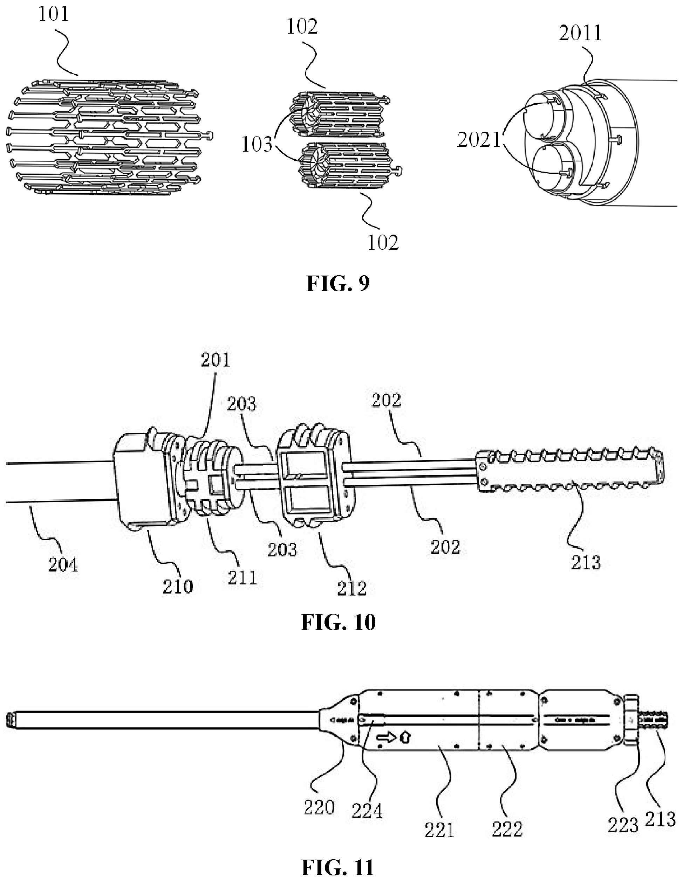

As shown in FIG. 10, the central assembly includes an outer sheath 204, a distal movable slider 210, a main stent fixing tube 201, a fixing block 211, a stepped tube 203, a proximal movable slider 212, an inner stent fixing member 202, and a movable screw rod 213.

The distal end of the main stent fixing tube 201 is provided with a first lug groove 2011 cooperating with a first lug structure 1011. The first lug structure 1011 is configured to cooperate with the first lug groove 2011 to secure the main stent 101 in the collapsed state. The proximal end of the main stent fixing tube 201 is secured to the fixing block 211.

The number of inner stent fixing members 202 is the same as the number of inner stents 102. The distal end of the inner stent fixing member 202 is provided with a second lug groove 2021 cooperating with a second lug structure 1021. The second lug structure 1021 is configured to cooperate with the second lug groove 2021 to secure the inner stent 102 in the collapsed state. The proximal end of the inner stent fixing member 202 is secured to the movable screw rod 213 and is configured to move along with the movable screw rod 213.

The number of stepped tubes 203 is the same as the number of inner stent fixing members 202. Each stepped tube 203 is configured to accommodate one inner stent fixing member 202. The distal end of the stepped tube 203 is provided with a cavity for accommodating the inner stent 102 in the collapsed state. The proximal end 203 of the stepped tube 203 is secured to the proximal movable slider 212 and is configured to move along with the proximal movable slider 212. A part of the stepped tube 203 is accommodated in the cavity of the main stent fixing tube 101.

The cavity of the outer sheath 204 is configured to accommodate the main stent fixing tube 201. The distal end has a cavity for accommodating the main stent 101 in the collapsed state. The proximal end of the outer sheath 204 is secured to the distal movable slider 210 and is configured to move along with the distal movable slider 210.

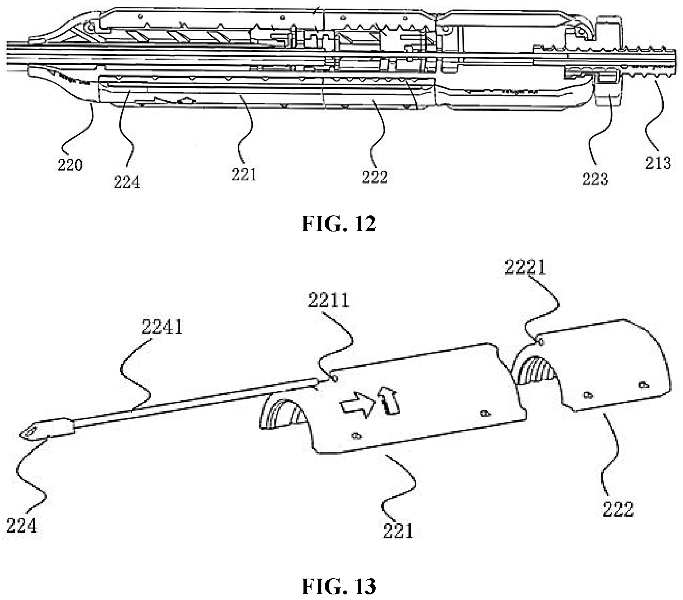

As shown in FIG. 11 and FIG. 12, the housing assembly includes a housing 220, a front rotary knob 221, a rear rotary knob 222, and a threaded rotary knob 223.

The central assembly is accommodated in the housing 220.

The front rotary knob 221 has an internal threaded structure. The internal threaded structure of the front rotary knob 221 is configured to cooperate with an external threaded structure of the distal movable slider 210. The front rotary knob 221 is configured to drive the distal movable slider to move axially through rotation.

The rear rotary knob 222 has an internal threaded structure. The internal threaded structure of the rear rotary knob 222 is configured to cooperate with an external threaded structure of the proximal movable slider 212. The rear rotary knob 222 is configured to drive the proximal movable slider 212 to move axially through rotation.

The threaded rotary knob 223 has an internal threaded structure. The internal threaded structure of the threaded rotary knob 223 is configured to cooperate with an external threaded structure of the movable screw rod 213. The threaded rotary knob 223 is configured to drive the movable screw rod 213 to move axially through rotation.

In the delivery device system of this example, the front rotary knob 221 is configured to drive the distal movable slider 210 to move axially to enable exposure or collapse of the main stent fixing tube 201 at a target position; and the rear rotary knob 222 is configured to drive the proximal movable slider 212 to move axially to enable exposure or collapse of the inner stent 102 at the target position. The front rotary knob 221 and the rear rotary knob 222 in the present application are controlled to rotate stepwise or synchronously to enable stepwise or synchronous deployment of the main stent 101 and the inner stent 102, thereby reducing displacement caused by deployment stress.

The housing assembly also includes a pin assembly 224 (referring to FIG. 13). The pin assembly 224 includes a pin body 2241. The front rotary knob 221 is provided with a first latching cavity 2211. The rear rotary knob 222 is provided with a second latching cavity 2221. The pin body 2241 is slidably disposed in the first latching cavity 2211 and the second latching cavity 2221 to enable synchronous rotation of the front rotary knob 221 and the rear rotary knob 222.

The thread pitch of the internal threaded structure of the front rotary knob 221 is equal to the thread pitch of the internal threaded structure of the rear rotary knob 222.

In this example, the front rotary knob 221 and the rear rotary knob 222 are rotated stepwise or synchronously to enable stepwise or synchronous deployment of the main stent 101 and the inner stent 102 with a short interval time, thereby reducing displacement caused by deployment stress.

### Embodiment five

This embodiment provides a delivery device system for a prosthetic mitral valve implant 100. This example is identical to embodiment four except that the thread pitch of the internal threaded structure of the front rotary knob 221 is greater than the thread pitch of the internal threaded structure of the rear rotary knob 222.

In this example, the thread pitch of the internal threaded structure of the front rotary knob 221 is greater than the thread pitch of the internal threaded structure of the rear rotary knob 222 to enable deployment of the main stent 101 and the inner stent 102 in sequence, thereby further reducing displacement caused by deployment stress.

In summary, the prosthetic mitral valve implant of the present application is configured to replace a native valve by at least two inner stents and corresponding biovalves, reducing the overall valve height and avoiding obstruction of the left ventricular outflow tract. Moreover, while conforming to the mitral annulus, the prosthetic mitral valve implant of the present application can avoid compression of the aortic root, thereby preventing impairment of the normal function of the aortic valve. In the delivery device system of the present application, the distal movable slider is driven to move axially through the front rotary knob to enable exposure or collapse of the main stent fixing tube at a target position; and the proximal movable slider is driven to move axially through the rear rotary knob to enable exposure or collapse of the inner stent at the target position. The front rotary knob and the rear rotary knob in the present application are controlled to rotate stepwise or synchronously to enable stepwise or synchronous deployment of the main stent and the inner stent, thereby reducing displacement caused by deployment stress.

The preceding are embodiments of the present application and are not intended to limit the protection scope of the present application. Those skilled in the art should understand that any changes or substitutions easily conceivable by those skilled in the art within the technical scope disclosed in the present application fall within the protection scope and the disclosed scope of the present application.

## Claims

1. A delivery device system for a prosthetic mitral valve implant, comprising a central assembly and a housing assembly, wherein
the central assembly comprises an outer sheath, a distal movable slider, a main stent fixing tube, a fixing block, a stepped tube, a proximal movable slider, an inner stent fixing member, and a movable screw rod; wherein
a proximal end of the main stent fixing tube is secured to the fixing block;
a proximal end of the inner stent fixing member is secured to the movable screw rod;
the stepped tube passes through the fixing block, a distal end of the stepped tube is provided with a cavity for accommodating an inner stent in a collapsed state, and a proximal end of the stepped tube is secured to the proximal movable slider; and a part of the stepped tube is accommodated in a cavity of the main stent fixing tube; and
a cavity of the outer sheath is configured to accommodate the main stent fixing tube, a distal end of the outer sheath has a cavity for accommodating a main stent in the collapsed state, and a proximal end of the outer sheath is secured to the distal movable slider; and
the housing assembly comprises a front rotary knob, a rear rotary knob, a threaded rotary knob, and a housing for housing the central assembly; wherein
the front rotary knob is configured to drive the distal movable slider to move axially through rotation;
the rear rotary knob is configured to drive the proximal movable slider to move axially through rotation;
the threaded rotary knob is configured to drive the movable screw rod to move axially through rotation; and
a thread pitch of an internal threaded structure of the front rotary knob is greater than or equal to a thread pitch of an internal threaded structure of the rear rotary knob.

2. The delivery device system of claim 1, wherein the housing assembly further comprises a pin assembly; wherein
the pin assembly comprises a pin body;
the front rotary knob and the rear rotary knob are each provided with a latching cavity; and
the pin body is slidably disposed in the latching cavity.

3. The delivery device system of claim 1, wherein a distal end of the main stent fixing tube is provided with a first lug groove cooperating with a first lug structure, and the first lug structure is configured to cooperate with the first lug groove to secure the main stent in the collapsed state.

4. The delivery device system of claim 1, wherein a number of inner stent fixing members is the same as a number of inner stents, a distal end of the inner stent fixing member is provided with a second lug groove cooperating with a second lug structure, and the second lug structure is configured to cooperate with the second lug groove to secure the inner stent in the collapsed state; and
a number of stepped tubes is the same as the number of inner stent fixing members, and each of the stepped tubes is configured to accommodate one of the inner stent fixing members.

5. The delivery device system of claim 1, wherein the front rotary knob has the internal threaded structure, and the internal threaded structure of the front rotary knob is configured to cooperate with an external threaded structure of the distal movable slider; and the front rotary knob is configured to drive the distal movable slider to move axially through rotation;
the rear rotary knob has the internal threaded structure, and the internal threaded structure of the rear rotary knob is configured to cooperate with an external threaded structure of the proximal movable slider; and the rear rotary knob is configured to drive the proximal movable slider to move axially through rotation; and
the threaded rotary knob has an internal threaded structure, and the internal threaded structure of the threaded rotary knob is configured to cooperate with an external threaded structure of the movable screw rod; and the threaded rotary knob is configured to drive the movable screw rod to move axially through rotation.

6. The delivery device system of claim 1, wherein the prosthetic mitral valve implant applicable to the delivery device system comprises the main stent, at least two inner stents disposed on an inner side of the main stent, and biovalves connected to the at least two inner stents; wherein
along an axial direction of the main stent, the main stent comprises a main stent flange structure at a distal end of the main stent, a first lug structure at a proximal end of the main stent, and a main stent body segment connecting the main stent flange structure and the first lug structure;
along an axial direction of an inner stent of the at least two inner stents, the inner stent comprises a second lug structure at a proximal end of the inner stent and an inner stent body segment connected to the second lug structure;
each of the at least two inner stents is provided with one of the biovalves; and
a stent fabric is disposed in a gap between the main stent and the at least two inner stents.

7. The delivery device system of claim 6, wherein in an expanded state, the main stent body segment has a height of 6-30 mm.

8. The delivery device system of claim 6, wherein in an expanded state, the inner stent body segment has a height of 6-40 mm.

9. The delivery device system of claim 6, wherein in the collapsed state, a cross-sectional area of the at least two inner stents is 40-90% of a cross-sectional area of the main stent.

10. The delivery device system of claim 6, wherein a barb structure is disposed on an outer side of the main stent body segment.
